Europäisches Patentamt
European Patent Office
Office européen des brevets

(11) Publication number: **0 094 833 A2**

(12) **EUROPEAN PATENT APPLICATION**

(21) Application number: 83302789.9

(22) Date of filing: 17.05.83

(51) Int. Cl.³: C 07 D 261/20
C 07 D 413/12, A 61 K 31/41
A 61 K 31/445

(30) Priority: 18.05.82 JP 84501/82

(43) Date of publication of application: 23.11.83 Bulletin 83/47

(84) Designated Contracting States: DE FR GB IT

(71) Applicant: Dainippon Pharmaceutical Co., Ltd.
25, Doshomachi 3-chome Higashi-ku
Osaka-shi, Osaka 541(JP)

(72) Inventor: Naruto, Shunsuke
7-18 Asukano-Kita 2-chome
Ikoma-shi Nara-ken(JP)

(72) Inventor: Kadokawa, Toshiaki
4-3 Okaminami-cho
Hirakata-shi Osaka-fu(JP)

(72) Inventor: Kawashima, Katzuyoshi
7-21 Eiraku-cho 3-chome
Suma-ku Kobe-shi Hyogo-ken(JP)

(74) Representative: Paget, Hugh Charles Edward et al,
MEWBURN ELLIS & CO. 2/3 Cursitor Street
London EC4A 1BQ(GB)

(54) **Novel 1,2-benzisoxazole derivatives.**

(57) Novel 1,2-benzisoxazole derivatives of the formula:

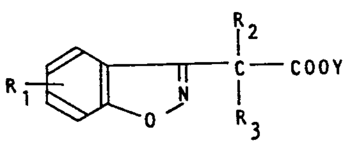

(I)

wherein $R_1$ is hydrogen atom, a halogen atom, hydroxy group, a lower alkyl group, trifluoromethyl group, or a lower alkoxy group, $R_2$ is hydroxy group, hydroxymethyl group, a cycloalkyl group, phenoxy group, phenylthio group, or benzyl group, $R_3$ is hydrogen atom or benzyl group, Y is a group of the formula:

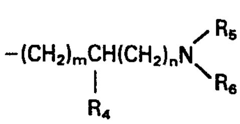

or

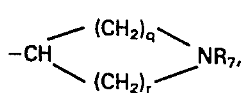

$R_4$ is hydrogen atom or a lower alkyl group, $R_5$ and $R_6$ are the same or different and are each a lower alkyl group or combine together with the adjacent nitrogen atom to form a heterocyclic group, $R_7$ is a lower alkyl group, m and n are the same or different and are each an integer of 0 to 3, sum of m and n is an integer of 1 to 4, and q and r are the same or different and are each an integer of 1 to 3, and sum of q and r is an integer of 3 to 5, and a pharmaceutically acceptable acid addition salt or quaternary ammonium salt thereof, said compounds having excellent antispasmodic activities, particularly a combination of anti-cholinergic activity and musculotropic activity, and being useful as an antispasmodic agent, and a pharmaceutical composition containing said compounds (I) or their salts as an active ingredient.

EP 0 094 833 A2

0094833

# NOVEL 1,2-BENZISOXAZOLE DERIVATIVES

The present invention relates to novel 1,2-benzisoxazole derivatives having antispasmodic activities, and a pharmaceutical composition containing the compound as an active ingredient.

The present invention provides compounds of the formula:

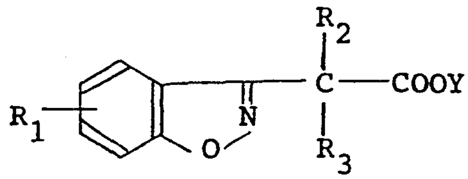

(I)

wherein $R_1$ is hydrogen atom, a halogen atom, hydroxy group, a lower alkyl group, trifluoromethyl group, or a lower alkoxy group, $R_2$ is hydroxy group, hydroxymethyl group, a cycloalkyl group, phenoxy group, phenylthio group, or benzyl group, $R_3$ is hydrogen atom or benzyl group, Y is a group of the formula: 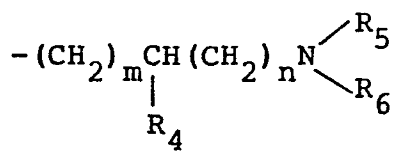 or 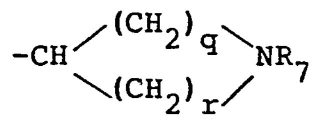 , $R_4$ is hydrogen atom or a lower alkyl group, $R_5$ and $R_6$ are the same or different and are each a lower alkyl group or combine together with the adjacent nitrogen atom to form a heterocyclic group, $R_7$ is a lower alkyl group, m and n are the same or different and are each an integer of 0 to 3, sum of m and n is an integer of 1 to 4, and q and r are the same or different and are each an integer of 1 to 3, and sum of q and r is an integer of 3 to 5, and a pharmaceutically acceptable acid addition salt or quaternary ammonium salt thereof.

The pharmaceutically acceptable quaternary ammonium salts of the compounds (I) are represented by the following formula:

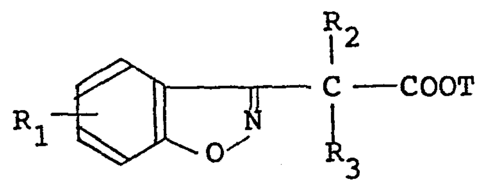

(I')

wherein T is a group of the formula: 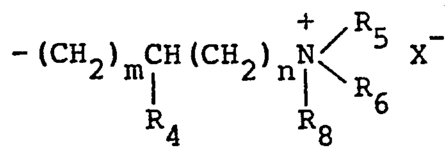

or 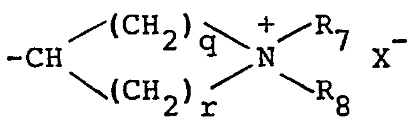, $R_8$ is a lower alkyl group, $X^-$ is a pharmaceutically acceptable anion, and $R_1$, $R_2$, $R_3$, $R_4$, $R_5$, $R_6$, $R_7$, m, n, q, and r are the same as defined above.

The compounds of the formula (I), and an acid addition salt or quaternary ammonium salt thereof contain optionally one or more asymmetrical carbons and further have geometrical isomeric structure, and hence, includes various stereo isomers. The present invention includes these stereoisomers, a mixture thereof, and a racemic mixture.

In the present specification and claims, the term "halogen atom" denotes fluorine, chlorine, bromine and iodine. The term "lower alkyl group" denotes a straight or branched alkyl group having 1 to 3 carbon atoms, such as methyl, ethyl, propyl or isopropyl, preferably methyl or ethyl. The term "lower alkoxy group" denotes a straight or branched alkoxy group having 1 to 3 carbon atoms, such as methoxy, ethoxy, propoxy or isopropoxy, preferably methoxy. The term "cycloalkyl group" denotes cyclopentyl, cyclohexyl

and cycloheptyl, preferably cyclohexyl. The term "heterocyclic group" which is formed by combining $R_5$ and $R_6$ groups together with the adjacent nitrogen atom denotes a 5- or 6-membered heterocyclic group which may contain additional one hetero atom selected from oxygen and sulfur, and includes 1-pyrrolidinyl, piperidino, hexamethylene-imino, morpholino and thiomorpholino. The "anion" represented by $X^-$ denotes an anion formed by removing a proton from a pharmaceutically acceptable and strong inorganic or organic acid and includes a halide ion (e.g. chloride, bromide or iodide ion), a lower alkylsulfate ion (e.g. methylsulfate or ethylsulfate ion), a lower alkylsulfonate ion (e.g. methanesulfonate ion), a substituted or unsubstituted benzenesulfonate ion (e.g. benzenesulfonate or p-toluenesulfonate ion), nitrate ion, preferably bromide or iodide ion.

Preferred compounds of this invention are as follows:

3-Diethylaminopropyl α-cyclohexyl-1,2-benzisoxazole-3-acetate or an acid addition salt thereof

3-Diethylaminopropyl α-cyclohexyl-5-chloro or methyl-1,2-benzisoxazole-3-acetate or an acid addition salt thereof

2-Diethylamino-1-methylethyl α-cyclohexyl-1,2-benzisoxazole-3-acetate or an acid addition salt thereof

2-Diethylamino-1-methylethyl α-cyclohexyl-5-chloro or methyl-1,2-benzisoxazole-3-acetate or an acid addition salt thereof

1-Methyl-3 or 4-piperidyl α-cyclohexyl-1,2-benzisoxazole-3-acetate or an acid addition salt thereof

1-Methyl-3 or 4-piperidyl α-cyclohexyl-5-chloro or methyl-1,2-benzisoxazole-3-acetate or an acid addition salt thereof

1,1-Dimethyl-3 or 4-[2-(1,2-benzisoxazol-3-yl)-2-cyclohexylacetoxy]piperidinium salt

1-Ethyl-3 or 4-piperidyl α-cyclohexyl-1,2-benzisoxazole-3-acetate or an acid addition salt thereof

1-Ethyl-3 or 4-piperidyl α-cyclohexyl-5-chloro or methyl-1,2-benzisoxazole-3-acetate or an acid addition salt thereof

1-Ethyl-1-methyl-3 or 4-[2-(1,2-benzisoxazol-3-yl)-2-cyclohexylacetoxy]piperidinium salt

2-Diethylaminoethyl α-cyclohexyl-1,2-benzisoxazole-3-acetate or an acid addition salt thereof

2-Diethylaminoethyl α-cyclohexyl-5-chloro or methyl-1,2-benzisoxazole-3-acetate or acid addition salt thereof

2-Piperidinoethyl α-cyclohexyl-1,2-benzisoxazole-3-acetate or an acid addition salt thereof

2-Piperidinoethyl α-cyclohexyl-5-chloro or methyl-1,2-benzisoxazole-3-acetate or an acid addition salt thereof

1-Methyl-4-piperidyl α-benzyl-1,2-benzisoxazole-3-acetate or an acid addition salt thereof

1-Methyl-4-piperidyl α-benzyl-5-chloro or methyl-1,2-benzisoxazole-3-acetate or an acid addition salt thereof

1-Methyl-4-piperidyl α,α-dibenzyl-1,2-benzisoxazole-3-acetate or an acid addition salt thereof

1-Methyl-4-piperidyl α,α-dibenzyl-5-chloro or methyl-1,2-benzisoxazole-3-acetate or an acid addition salt thereof

1-Methyl-4-piperidyl α-phenylthio-1,2-benzisoxazole-3-acetate or an acid addition salt thereof

1-Methyl-4-piperidyl α-phenylthio-5-chloro or methyl-1,2-benzisoxazole-3-acetate or an acid addition salt thereof

Although known antispasmodic agents have no 1,2-benzisoxazole nucleus in the chemical structure thereof, the compounds of the present invention have characteristically 1,2-benzisoxazole nucleus in the molecule thereof and show excellent pharmacological activities suitable for using as an antispasmodic agent.

The compounds of the present invention have relatively potent anti-cholinergic activity (to be sometimes referred to as "anti-ACh activity"). As shown in Table 1 hereinafter, the anti-ACh activity of several compounds of this invention is stronger than or nearly equal to that of scopolamine-N-butyl bromide.

The present invention is further characteristic in that the several compounds of this invention show their antispasmodic effects through a combination of anti-ACh activity and musculotropic activity (to be sometimes referred to as "anti-KCl activity"). Moreover, these two

activities of the several compounds of this invention are unexpectedly balanced. As mentioned by Toson et al [cf. Arzneim.-Forsch., 28 (II), Heft 7, 1130 (1978)], an antispasmodic agent with balanced anti-cholinergic and musculotropic activities exerts antispasmodic effect with less undesirable side effects, such as mydriasis, inhibition of salivary secretion and tachycardia, than the pure anti-cholinergic agent and with less cardiovascular effect than the typical smooth muscle-relaxant such as papaverine.

It has been reported that rociverine is the first antispasmodic agent with balanced anti-cholinergic and musculotropic activity [cf. Arzneim.-Forsch., 28, (II), Heft 7, 1130 (1978)]. As shown in Table 2 hereinafter, several compounds of this invention exhibit both anti-ACh and Anti-KCl activities. These two activities of the several compounds of this invention appear to be balanced, and are stronger than those of rociverine.

In respect to antispasmodic agents having such both of anti-cholinergic activity and musculotropic activity, it may be expected that the therapeutic scope of the compounds as an antispasmodic agent will be extended.

Preferred compounds having these both activities are as follows:

3-Diethylaminopropyl α-cyclohexyl-1,2-benzisoxazole-3-acetate or its acid addition salt

2-Diethylaminoethyl α-cyclohexyl-1,2-benzisoxazole-3-acetate or its acid addition salt

2-Diethylamino-1-methylethyl α-cyclohexyl-1,2-benzisoxazole-3-acetate or its acid addition salt

2-Piperidinoethyl α-cyclohexyl-1,2-benzisoxazole-3-acetate or its acid addition salt

1-Methyl-4-piperidyl α-cyclohexyl-5-chloro-1,2-benzisoxazole-3-acetate or its acid addition salt

2-Diethylaminoethyl α-cyclohexyl-5-chloro-1,2-benzisoxazole-3-acetate or its acid addition salt

1-Methyl-4-piperidyl α,α-dibenzyl-1,2-benzisoxazole-3-acetate or its acid addition salt

among which the first-mentioned compound is particularly preferable.

The 1,2-benzisoxazole derivatives of the formula (I) can be prepared, for example, by reacting a compound of the formula:

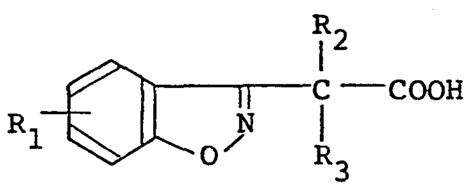 (II)

wherein $R_1$, $R_2$ and $R_3$ are the same as defined above, or its reactive derivative at the carboxyl group with a compound of a formula:

Z-Y (III)

wherein Z is hydroxy group or a residue of a reactive ester, and Y is the same as defined above.

The methods for the preparation of the compounds (I) are explained in more detail below.

Process-A

The compound of formula (I) wherein $R_2$ is hydroxy

group or hydroxymethyl group, that is, a compound of the formula:

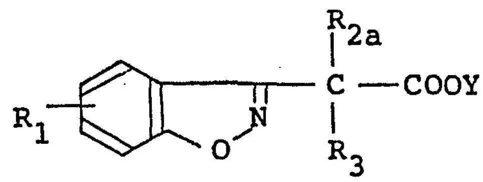

(I-A)

wherein $R_1$, $R_3$ and Y are the same as defined above, and $R_{2a}$ is hydroxy group or hydroxymethyl group, is prepared by reacting a compound of the following formula:

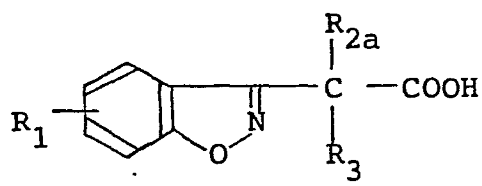

(II-a)

wherein $R_1$, $R_{2a}$ and $R_3$ are the same as defined above, or a metal salt thereof with a compound of the formula:

halogen-Y (III-a)

wherein Y is the same as defined above.

The reaction of the above Process-A is usually carried out in a solvent such as lower alcohols (e.g. isopropyl alcohol, sec-butyl alcohol) and ethers (e.g. tetrahydrofuran, dioxane), among which isopropyl alcohol is preferable. The solvent is preferably used in an amount of as small as possible. The reaction temperature and reaction period of time may vary with kinds of the starting materials, but the reaction is usually carried out at a temperature of from room temperature to about 100°C for about 1 to 20 hours.

Process-B

The compound of the formula (I) wherein $R_2$ is a cycloalkyl group, phenoxy group, phenylthio group or benzyl

group and $R_3$ is hydrogen atom, that is, a compound of the formula:

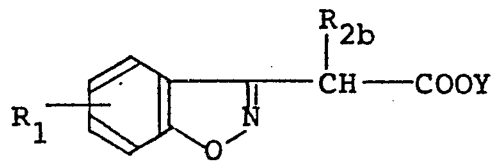 (I-B)

wherein $R_1$ and Y are the same as defined above, and $R_{2b}$ is a cycloalkyl group, phenoxy group, phenylthio group or benzyl group, is prepared by reacting a mixed anhydride derivative or an active ester derivative of a compound of the formula:

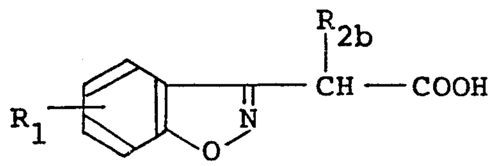 (II-b)

wherein $R_1$ and $R_{2b}$ are the same as defined above, with a compound of the following formula:

HO-Y (III-b)

wherein Y is the same as defined above.

In the above Process-B, the carboxylic acid compound (II-b) is firstly converted into a mixed anhydride or activated ester by a conventional method and then reacted with the compound (III-b). The reaction is usually carried out in a solvent and optionally in the presence of a base (e.g. tertiary amines or alkali metal carbonates). Suitable examples of the solvent are aromatic hydrocarbons such as benzene, toluene or xylene; ethers such as dioxane; halogenated hydrocarbons such as dichloromethane, chloroform or dichloroethane; acetonitrile; dimethylformamide; pyridine; or the like. The reaction is usually done at a temperature of about -20 to 150°C for about 1 to 24 hours.

Process-C

The compound of the formula (I) wherein $R_2$ is $R_{2b}$ and $R_3$ is benzyl group, that is, compound of the formula:

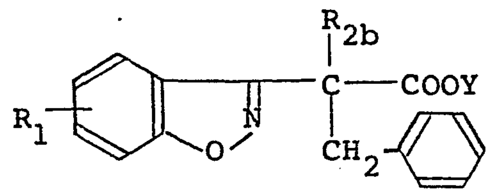

(I-C)

wherein $R_1$, $R_{2b}$ and Y are the same as defined above, are obtained by reacting a compound of the following formula:

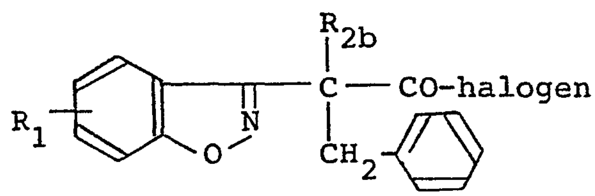

(II-c)

wherein $R_1$ and $R_{2b}$ are the same as defined above, with a compound of the formula (III-b) as defined in process-B.

The reaction is usually carried out in the absence or presence of a solvent and optionally in the presence of a base (e.g. tertiary amines or alkali metal carbonates). Suitable examples of the solvent are aromatic hydrocarbons such as benzene, toluene or xylene; ethers such as dioxane; halogenated hydrocarbons such as dichloromethane, chloroform or dichloroethane; or the like. The reaction is usually done at a temperature of about -20 to 150°C for about 1 to 24 hours.

The compounds (I) can be isolated from the reaction mixture and purified by a conventional method. The compound (I) is obtained in the form of a free base or an acid addition salt thereof depending on the kinds of the starting materials and the conditions of reaction and

subsequent procedures. When the compound (I) is obtained in the form of an acid addition salt, it may be converted into a free base by treating it with a base such as an alkali metal carbonate or ammonia. Besides, when the compound (I) is obtained in the form of a free base, it may be converted into an acid addition salt thereof by treating it with a pharmaceutically acceptable inorganic or organic acid. Suitable examples of the inorganic or organic acid are inorganic acids such as hydrochloric acid, hydrobromic acid, hydroiodic acid, phosphoric acid, nitric acid, sulfuric acid, or the like, and organic acids such as citric acid, fumaric acid, maleic acid, lactic acid, succinic acid, malic acid, tartaric acid, methanesulfonic acid, or the like.

When the compound (I) has one or more asymmetric carbon atoms, it is usually obtained in the form of a racemic mixture or a mixture of stereoisomers, but the mixture can be separated into each stereo-isomer by a conventional optical resolution, chromatographic or fractional crystallization method.

The quaternary ammonium salts of the compounds (I), that is, compounds (I') can be prepared by reacting a compound of the formula (I) with a compound of the formula (IV):

$$R_8—X \qquad (IV)$$

wherein $R_8$ is as defined above, and X is a residue of an anion as represented above by $X^-$.

The compound of the formula (IV) includes alkyl

halides, dialkyl sulfates. Suitable examples of the alkyl halides are methyl iodide, methyl bromide, methyl chloride, ethyl iodide, ethyl bromide, ethyl chloride, propyl iodide, propyl bromide, isopropyl iodide, isopropyl bromide, butyl iodide, butyl bromide, isobutyl bromide, or the like. Suitable examples of the dialkyl sulfates are dimethyl sulfate, diethyl sulfate, or the like.

The reaction of the compound (I) with the compound (IV) can be carried out by a conventional method which is usually used in the preparation of conventional quaternary ammonium salts, for example, by reacting both compounds in the absence or presence of a solvent. Suitable examples of the solvent are aromatic hydrocarbons such as benzene, toluene or xylene; ethers such as tetrahydrofuran or dioxane; dialkyl ketones such as acetone, methyl ethyl ketone or methyl isobutyl ketone; lower alcohols such as methanol, ethanol, isopropyl alcohol; acetonitrile; dimethylformamide; or a mixture thereof. The reaction temperature may vary with the kinds of the compound (I), compound (IV) and reaction solvent, but is usually in the range of about 10 to 130°C, and the reaction period is usually in the range of about 10 minutes to 72 hours.

When a compound of the formula (I) is reacted with a compound of the formula (IV) wherein $R_8$ is a group different from the group $R_7$, two different quaternary ammonium salts are produced in a ratio of about 1 : 1 by mole. In the present specification, these two different

isomers are defined as follows: the quaternary ammonium salt wherein one of the groups $R_7$ and $R_8$ which has the higher priority determined by the sequence rules of Cahn, Ingold and Prelog [cf. R.S. Cahn et al., Angew. Chem. Intern. Ed. Engl., 5, 385 (1966)] is in the trans-configuration to 2-$R_2$-2-$R_3$-2-(1,2-benzisoxazol-3-yl)acetoxy group is defined as $\alpha$-isomer, and another quaternary ammonium salt is defined as $\beta$-isomer. Thus, when $R_7$ and $R_8$ in the formula (I') are methyl and ethyl group, respectively, the quaternary ammonium salt wherein the signal of methyl group bonded to the quaternary nitrogen atom appears at the higher field in the $^1$H-NMR spectra is defined as $\alpha$-isomer, and the salt wherein the signal appears at the lower field is defined as $\beta$-isomer. The separation of the $\alpha$-isomer and $\beta$-isomer can be carried out by chromatography such as high performance liquid chromatography, or fractional crystallization.

Among the starting compounds of the formulae (II-a), (II-b) and (II-c), the compound of the formula (II-b) wherein $R_1$ is hydrogen atom and $R_{2b}$ is a group other than phenylthio group is novel. The novel compound can be prepared by treating 2-(1,2-benzisoxazol-3-yl)acetic acid derivative [cf. J. Heterocyclic Chem., 8, 397 (1971)] in the same manner as described in Reference Examples hereinafter. Besides, the compounds of the formula (II-b) wherein $R_1$ is hydrogen and $R_{2b}$ is phenylthio group can be prepared by the method as disclosed in Chem. Pharm. Bull., 30(4), 1175, (1982).

Following Experiments 1 to 3 show the pharmacological activities of the compounds of the present invention in comparison with reference compounds.

[Antispasmodic activity *in vitro*]

Experiment 1 Inhibitory effect on contractile response induced by acetylcholine (Anti-ACh)

From male Hartley strain guinea pig, weighing 300 - 350 g, section of the ileum about 3 cm in length was prepared and mounted in Tyrode's solution kept at 35°C and oxygenated with 95% $O_2$ - 5% $CO_2$. The response of the ileum to acetylcholine (2 X $10^{-8}$ g/ml) was recorded isotonically with an initial tension of 1 g. Test compounds were applied to 2 minutes before adding acetylcholine.

Each compound was tested in three different concentrations. On the basis of the percent inhibition in each concentration, $IC_{50}$ value, i.e., the concentration required for 50% inhibition of the response induced by acetylcholine, was determined by a usual graphic method.

The results of the Experiment 1 are shown in Table 1.

Table 1 Antispasmodic activity *in vitro*

| Compound* | Anti-ACh ($IC_{50}$ : g/ml) |
|---|---|
| 1 | $1.6 \times 10^{-7}$ |
| 2 | $3.4 \times 10^{-7}$ |
| 3 | $1.2 \times 10^{-7}$ |
| 4 | $4.2 \times 10^{-7}$ |
| 5 | $3.7 \times 10^{-8}$ |
| 6 | $4.9 \times 10^{-8}$ |
| 7 | $6.9 \times 10^{-7}$ |
| 8 | $4.8 \times 10^{-7}$ |
| 9 | $4.1 \times 10^{-7}$ |
| 10 | $8.7 \times 10^{-7}$ |
| 24 | $6.6 \times 10^{-7}$ |
| 28 | $6.5 \times 10^{-7}$ |
| 29 | $9.6 \times 10^{-7}$ |
| 31 | $1.4 \times 10^{-8}$ |
| 32 | $2.1 \times 10^{-7}$ |
| 33 | $1.3 \times 10^{-8}$ |
| 34 | $2.6 \times 10^{-8}$ |
| 35 | $5.7 \times 10^{-8}$ |
| Atropine sulfate | $5.0 \times 10^{-9}$ |
| Scopolamine-N-butyl bromide | $8.9 \times 10^{-8}$ |
| Papaverine hydrochloride | $1.3 \times 10^{-5}$ |
| Rociverine** | $1.1 \times 10^{-6}$ |

Note

* The figure means the number of working Examples disclosed hereinafter for preparing the test compound.

** Rociverine

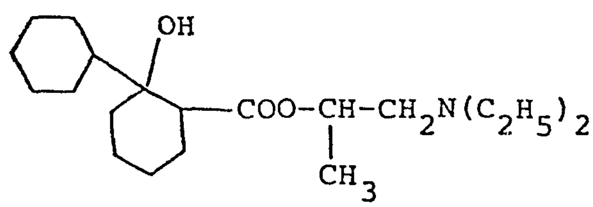

As shown in Table 1,

(1) the compounds of the present invention have relatively potent anti-ACh activity,

(2) the anti-ACh activity of the compounds of this invention is stronger than that of rociverine, and nearly equal to that of scopolamine-N-butyl bromide, while somewhat weaker than that of atropine sulfate.

Experiment 2 Inhibitory effect on contractile response induced by potassium chloride (Anti-KCl)

In the same manner as described in the above Experiment 1, isolated guinea pig taenia-coli about 3 cm in length was prepared, and mounted in Tyrode's solution. The contractile response was measured with an isometric transducer and recorded on recticorder. Each preparation was adjusted to a tension of 1g and allowed to equilibrate for 30 minutes before measuring the effects of the test compounds. Test compounds were applied to 5 minutes before adding potassium chloride (40 mM). Each compound was tested in 2 - 3 concentrations. On the basis of the percent inhibition in each concentration, $IC_{50}$ value, i.e., the concentration required for 50% inhibition of the response induced by potassium chloride, was determined by a usual graphic method. The results are shown in Table 2.

Table 2 Antispasmodic activity *in vitro*

| Compound *1 | $IC_{50}$ (g/ml) | |
|---|---|---|
| | Anti-KCl | Anti-ACh *2 |
| 1 | $8.5 \times 10^{-7}$ | $1.6 \times 10^{-7}$ |
| 2 | $3.2 \times 10^{-6}$ | $3.4 \times 10^{-7}$ |
| 3 | $1.0 \times 10^{-6}$ | $1.2 \times 10^{-7}$ |
| 4 | $1.6 \times 10^{-6}$ | $4.2 \times 10^{-7}$ |
| 9 | $2.7 \times 10^{-6}$ | $4.1 \times 10^{-7}$ |
| 10 | $3.3 \times 10^{-6}$ | $8.7 \times 10^{-7}$ |
| 28 | $5.6 \times 10^{-7}$ | $6.5 \times 10^{-7}$ |
| Papaverine hydrochloride | $1.3 \times 10^{-6}$ | $1.3 \times 10^{-5}$ |
| Rociverine | $5.4 \times 10^{-6}$ | $1.1 \times 10^{-6}$ |

Note

*1 The figure means the number of working Examples disclosed hereinafter for preparing the test compound.

*2 Reference data (see Table 1).

As shown in Table 2, the anti-KCl activity of the compounds of the present invention is stronger than or neary equal to that of rociverine.

[Antispasmodic activity *in vivo*]

*Experiment 3* Inhibitory effect on spontaneous gastric motility (Anti-SGM)

This effect was examined using male Wistar strain rats or male Albino strain rabbits, with chronically implanted strain gauge force transducer. The rats or rabbits were allowed to stand for 3 - 5 days for recovery from the surgery and fasted for 18 hours before experiment. Then, spontaneous gastric motility in conscious rats or rabbits was recorded and the sum of amplitude of each contraction was calculated every 15 minutes. Inhibitory effect of test compound by oral administration was expressed as a maximum percent inhibition of initial spontaneous motility. The results are shown in Table 3.

Table 3 Antispasmodic activity *in vivo*

| Compound | Animal | Dose (mg/kg) | Route | Inhibition (%) |
|---|---|---|---|---|
| 1* | Rat | 100 | po** | 36 |
| | | 200 | po | 49 |
| | Rabbit | 100 | po | 52 |

Note

* The compound prepared by working Example 1.

** po : oral administration

The compounds of the formula (I) and pharmaceutically acceptable acid addition salts or quaternary ammonium salts thereof are useful as an antispasmodic agent for the treatment of peptic ulcer and various hyperactive symptoms in gastrointestinal tract, for example, spastic constipation, irritable diarrhea, cardiospasm and pylorospasm in mammals including human being.

The compounds (I) and pharmaceutically acceptable acid addition salts or quaternary ammonium salts thereof can be administered by oral, parenteral or intrarectal route, preferably by oral route. The clinical dose of the compound (I) and pharmaceutically acceptable acid addition salts or quaternary ammonium salts thereof may vary according to the kinds of the compound,

administration routes, severity of disease, age of patients, or the like, but is usually in the range of 0.05 to 40 mg per kg of body weight per day, preferably 0.1 to 20 mg per kg of body weight per day, in human. The dose may be divided and administered in two to several times per day.

The compounds (I) and pharmaceutically acceptable acid addition salts or quaternary ammonium salts thereof are usually administered to patients in the form of a pharmaceutical composition which contains a non-toxic and effective amount of the compounds. The pharmaceutical composition is usually prepared by admixing the active compounds (I) or their salts with conventional pharmaceutical carrier materials, such as lactose, glucose or the like, which are unreactive with the active compounds (I) or their salts.

The pharmaceutical composition may be in the dosage form of tablets, capsules, granules, fine granules, powders, syrups, suspension, suppositories, injections, or the like. These preparations may be prepared by conventional methods.

The present invention is illustrated more specifically by the following Examples and Reference Examples. It should be understood that the invention is not limited to these examples. The compounds were identified by elemental analysis, mass spectrum, infrared spectrum, nuclear magnetic resonance (NMR) spectrum, etc.

Reference Example 1

α-Cyclohexyl-1,2-benzisoxazole-3-acetic acid;

50% Sodium hydride (4.6 g) was added portionwise at 0°C to a solution of methyl 1,2-benzisoxazole-3-acetate (20 g) in dimethylformamide (60 ml). The mixture was stirred for 30 min at room temperature. Cyclohexyl iodide (24 g) was added to the mixture. After stirring for 30 min at room temperature, toluene (500 ml), water (200 ml) and concentrated hydrochloric acid (10 ml) were added to the reaction mixture. After shaking the mixture, the toluene layer was washed with water, dried over anhydrous sodium sulfate and evaporated in vacuo. The residue was applied on a column of silica gel (100 g) using chloroform as a developing solvent. The first eluate of yellow material was discarded. The second eluate gave an oil (14.7 g) which was applied on a column of silica gel (80 g). The first fraction of toluene eluate was evaporated in vacuo to give an oily methyl α-cyclohexyl-1,2-benzisoxazole-3-acetate (6.4 g).

A mixture of methyl α-cyclohexyl-1,2-benzisoxazole-3-acetate (6.3 g), ethanol (70 ml), sodium hydroxide (1.4 g) and water (8 ml) was heated at 60°C for 30 min. After evaporation of ethanol in vacuo, toluene and water were added to the residue. After shaking the mixture, the aqueous layer was made acidic by addition of diluted hydrochloric acid, and extracted with ethyl acetate. The ethyl acetate layer was dried over anhydrous sodium sulfate and evaporated in vacuo to give α-cyclohexyl-1,2-benzisoxazole-3-acetic acid (5 g). Recrystallization from a mixture of ethyl acetate and hexane gave an analytical sample, colorless crystals, m.p. 135-137°C.

Reference Example 2

α-Cyclohexyl-5-chloro-1,2-benzisoxazole-3-acetic acid:

An oily methyl α-cyclohexyl-5-chloro-1,2-benzisoxazole-3-acetate (2.8 g) was obtained in substantially the same manner as described in Reference Example 1, using methyl 5-chloro-1,2-benzisoxazole-3-acetate (11 g), dimethylformamide (110 ml), 50% sodium hydride (2.4 g) and cyclohexyl iodide (21 g).

α-Cyclohexyl-5-chloro-1,2-benzisoxazole-3-acetic acid (1.95 g) was obtained in substantially the same manner as described in Reference Example 1, using methyl α-cyclohexyl-5-chloro-1,2-benzisoxazole-3-acetate (2.44 g). Recrystallization from a mixture of diethyl ether and hexane gave an analytical sample, colorless crystals, m.p. 150-152°C.

Reference Example 3

α-Benzyl-1,2-benzisoxazole-3-acetic acid:

60% Sodium hydride (2.3 g) was added portionwise at 0°C to a solution of methyl 1,2-benzisoxazole-3-acetate (10 g) in dimethylsufoxide (100 ml). The mixture was stirred for 30 min at room temperature. Benzyl bromide (9.85 g) was added to the mixture. After stirring for 2 hr at room temperature, the reaction mixture was diluted with water and extracted with chloroform. The chloroform layer was washed with water, dried over anhydrous sodium sulfate,

and evaporated *in vacuo*. The oily residue was applied on a column of silica gel. The chloroform-hexane (1 : 1) eluate afforded an oily methyl α-benzyl-1,2-benzisoxazole-3-acetate (9.6 g).

A mixture of methyl α-benzyl-1,2-benzisoxazole-3-acetate (7.6 g), dioxane (120 ml), and 10% hydrochloric acid (40 ml) was refluxed for 6 hr, and evaporated *in vacuo*. Dichloromethane and 1% sodium hydroxide were added to the residue, and the mixture was shaken. The aqueous layer was made acidic by addition of diluted hydrochloric acid, and extracted with dichloromethane. The dichloromethane layer was dried over anhydrous sodium sulfate and evaporated *in vacuo*. The residue was recrystallized from a mixture of diethyl ether and hexane to give α-benzyl-1,2-benzisoxazole-3-acetic acid (5.5 g), m.p. 84-85°C.

Reference Example 4

α,α-Dibenzyl-1,2-benzisoxazole-3-acetic acid:

60% Sodium hydride (6.9 g) was added portionwise at 0°C to a solution of methyl 1,2-benzisoxazole-3-acetate (15 g) in dimethylsulfoxide (100 ml). The mixture was stirred for 30 min at room temperature. Benzyl bromide (29.6 g) was added to the mixture. After stirring for 4 hr at room temperature, the reaction mixture was diluted with water and extracted with chloroform. The chloroform layer was washed with water, dried over anhydrous sodium sulfate, and evaporated *in vacuo*. The oily residue (37 g) was applied on a column of silica gel. The chloroform-hexane

(3:1) eluate gave methyl α,α-dibenzyl-1,2-benzisoxazole-3-acetate (15.5 g). Recrystallization from ethanol gave an analytical sample, colorless crystals, m.p. 82-84°C.

α,α-Dibenzyl-1,2-benzisoxazole-3-acetic acid (10.5 g) was obtained in substantially the same manner as in Reference Example 1, using methyl α,α-dibenzyl-1,2-benzisoxazole-3-acetate (13 g). Recrystallization from a mixture of diethyl ether and hexane gave an analytical sample, colorless crystals, m.p. 195-196.5°C.

Reference Example 5

α-Phenoxy-1,2-benzisoxazole-3-acetic acid:

50% Sodium hydride (2.06 g) and α-bromo-1,2-benzisoxazole-3-acetic acid (5.0 g) were added in turn to a solution of phenol (2.25 g) in ethanol (20 ml). The mixture was refluxed for 6 hr. After evaporation of solvent *in vacuo*, the residue was dissolved in water and washed with chloroform. The aqueous layer was made acidic by addition of diluted hydrochloric acid and extracted with chloroform. The chloroform layer was dried over anhydrous sodium sulfate and evaporated *in vacuo*. The residue was recrystallized from toluene to give α-phenoxy-1,2-benzisoxazole-3-acetic acid (3.6 g), m.p. 148-149°C (dec.).

Reference Example 6

α-Hydroxy-1,2-benzisoxazole-3-acetic acid:

Potassium acetate (34 g) was added to a solution of 18-crown-6 (1 g) in acetonitrile (350 ml). The mixture

was stirred for 2 hr at room temperature. Methyl α-bromo-1,2-benzisoxazole-3-acetate (49.5 g) was added to the mixture. After stirring for 16 hr at room temperature, the reaction mixture was filtered, and the precipitate was washed with toluene. The acetonitrile and toluene filtrates were combined and evaporated _in vacuo_. The oily residue was applied on a column of silica gel (100 g). The toluene eluate, toluene-chloroform (1:1) eluate and colorless chloroform eluate were combined and evaporated to give an oily methyl α-acetoxy-1,2-benzisoxazole-3-acetate (45 g). The methyl ester thus obtained was dissolved in a mixture of 5% sodium carbonate (260 ml) and methanol (200 ml). The mixture was heated at 65°C for 1 hr, and methanol was evaporated. The residual solution was washed with toluene. The aqueous layer was made acidic by addition of diluted hydrochloric acid and extracted with ethyl acetate. The ethyl acetate layer was dried over anhydrous sodidum sulfate and evaporated. The residue was washed with hexane to give α-hydroxy-1,2-benzisoxazole-3-acetic acid (34 g). Recrystallization from a mixture of diethyl ether and hexane gave an analytical sample, colorless crystals, m.p. 119-122°C.

Reference Example 7

α-Benzyl-α-hydroxy-1,2-benzisoxazole-3-acetic acid:

A mixture of α-hydroxy-1,2-benzisoxazole-3-acetic acid (34 g), methanol (150 ml) and concentrated sulfuric

acid (0.5 ml) was refluxed for 6 hr. After evaporation of methanol, 5% sodium carbonate and toluene were added to the residue, and the mixture was shaken. The toluene layer was washed with water, dried over sodium sulfate and evaporated to give methyl α-hydroxy-1,2-benzisoxazole-3-acetate (34 g). Recrystallization from toluene gave an analytical sample, colorless crystals, m.p. 64-65°C.

Under the dried nitrogen stream and anhydrous condition, a solution of methyl α-hydroxy-1,2-benzisoxazole-3-acetate (4.2 g) in dried tetrahydrofuran (30 ml) was added dropwise for 2 hr at -60°C with stirring to a solution of lithium diisopropylamide which was prepared from diisopropylamine (4.4 g) and 15% butyl lithium hexane solution (25.6 ml) in dried tetrahydrofuran (30 ml) according to a usual method. The mixture was stirred for 1 hr at -65°C. A solution of benzyl bromide (3.8 g) in dried tetrahydrofuran (30 ml) was added to the mixture. The temperature of the reaction mixture was gradually raised to 18°C, and the mixture was allowed to stand overnight at 18°C. Toluene and diluted hydrochloric acid were added at 0°C with stirring to the reaction mixture to make acidic. The organic layer was dried over anhydrous sodium sulfate and evaporated <u>in vacuo</u> to give an oily residue which was applied on a column of silica gel. The column was eluted with toluene and chloroform in turn. The first chloroform eluate gave methyl α-benzyl-α-hydroxy-1,2-benzisoxazole-3-acetate (4 g). Recrystallization from diethyl ether-

hexane gave an analytical sample, colorless crystals, m.p. 91-93°C.

α-Benzyl-α-hydroxy-1,2-benzisoxazole-3-acetic acid (2.7 g) was obtained in substantially the same manner as in Reference Example 1, using methyl α-benzyl-α-hydroxy-1,2-benzisoxazole-3-acetate (2.8 g). Recrystallization from a mixture of ethyl acetate and hexane gave an analytical sample, colorless crystals, m.p. 103-105°C.

Reference Example 8

α-Hydroxymethyl-1,2-benzisoxazole-3-acetic acid:

A mixture of 1,2-benzisoxazole-3-acetic acid (10.5 g), sodium carbonate (12.6 g), water (120 ml) and 37 % formaldehyde solution (30 ml) was heated for 8 hr at 90°C. After being cooled, the reaction mixture was made acidic by addition of diluted hydrochloric acid and extracted with ethyl acetate. The organic layer was dried over anhydrous sodium sulfate and evaporated *in vacuo*. The residue was applied on a column of silica gel (60 g). The column was eluted with 6% methanol-chloroform. The fractions which showed a spot of Rf 0.15 on thin layer chromatogram (silicagel, 10% acetic acid-chloroform) were collected and evaporated *in vacuo*. The residue was recrystallized from a mixture of ethyl acetate and diethyl ether gave α-hydroxymethyl-1,2-benzisoxazole-3-acetic acid (4.3 g), m.p. 128-130°C.

Example 1

3-Diethylaminopropyl α-cyclohexyl-1,2-benzisoxazole-3-acetate hydrobromide:

p-Toluenesulfonyl chloride (0.8 g) was added to a solution of α-cyclohexyl-1,2-benzisoxazole-3-acetic acid (1 g) in toluene (20 ml). The mixture was stirred at 90°C for 2 hr. Triethylamine (1 ml) and 3-diethylaminopropanol (0.8 g) were added in turn to the mixture. After stirring overnight at room temperature, the reaction mixture was diluted with toluene, and then shaken with 5% sodium carbonate and water. The toluene layer was extracted with 5% hydrochloric acid. The aqueous layer was made alkaline with concentrated ammonium hydroxide and extracted with toluene. The toluene layer was dried over anhydrous sodium sulfate and evaporated in vacuo. The residue was converted into hydrobromide with hydrobromic acid. The hydrobromide was recrystallized from a mixture of ethanol and diethyl ether to give 3-diethylaminopropyl α-cyclohexyl-1,2-benzisoxazole-3-acetate hydrobromide (1.53 g), m.p. 128-130°C.

Example 2

1-Methyl-4-piperidyl α-cyclohexyl-5-chloro-1,2-benzisoxazole-3-acetate oxalate:

p-Toluenesulfonyl chloride (0.9 g) and triethylamine (0.6 ml) were added to a solution of α-cyclohexyl-5-chloro-1,2-benzisoxazole-3-acetic acid (1.2 g) in toluene (20 ml). The mixture was stirred for 1 hr at room temperature. 1-Methyl-4-hydroxypiperidine (1.8 ml) was added to the mixture, and then the mixtue was heated at 70°C for 3 hr. The reaction mixtue was treated in the same manner as in Example 1. The toluene layer thus

obtained was evaporated to give an oily residue (1.45 g). Oxalic acid dihydrate (0.47 g) was added to a solution of the residue in ethanol (10 ml), and then the mixture was evaporated. The residue was recrystallized from a mixture of ethanol and diethyl ether to give 1-methyl-4-piperidyl α-cyclohexyl-5-chloro-1,2-benzisoxazole-3-acetate oxalate (1.5 g), m.p. 200-203°C.

Examples 3 to 16

Various compounds listed in the following Table 4 were prepared in substantially the same manner as in Example 1 or Example 2, using the corresponding α-substituted 1,2-benzisoxazole-3-acetic acid and aminoalcohol.

Table 4

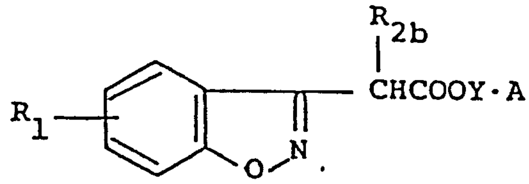

| Ex. | $R_1$ | $R_{2b}$ | Y | A | m.p. (°C) Recry. Solv.* |
|---|---|---|---|---|---|
| 3 | H | 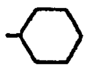 | $-(CH_2)_2N(C_2H_5)_2$ | HBr | 133-135 (A-E) |
| 4 | " | " | $-CH(CH_3)CH_2N(C_2H_5)_2$ | " | 136-138 (A-E) |
| 5 | " | " | 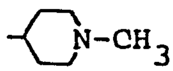 | " | 199-201 (A-E) |
| 6 | " | " | 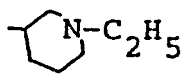 | $(CO_2H)_2$ | 103-106 (A-E) |
| 7 | " | " | $-CH(CH_3)(CH_2)_3N(C_2H_5)_2$ | HBr | 153-156 (A-E) |
| 8 | " | " | $-(CH_2)_4N(C_2H_5)_2$ | " | 119-121 (A-E) |
| 9 | " | " | $-(CH_2)_2$ 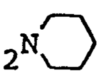 | $(CO_2H)_2$ | 179-181 (A-E) |
| 10 | 5-Cl | " | $-(CH_2)_2N(C_2H_5)_2$ | " | 153-154 (A-E) |
| 11 | H | $-CH_2Ph$ | 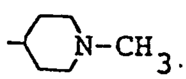 | " | 155-157 (A-H) |
| 12 | " | " | $-(CH_2)_2$ 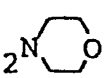 | " | 150-152 (A-H) |
| 13 | " | -OPh | $-(CH_2)_2$ 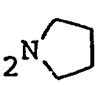 | HCl | 121-125 (A-H) |
| 14 | " | " | 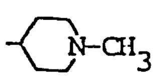 | " | 175-177 (A-H) |
| 15 | " | " | $-(CH_2)_2N(CH_3)_2$ | " | 151-153 (A-H) |
| 16 | " | -SPh | 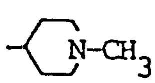 | --- | 65-67 ( H ) |

Ph : $C_6H_5$, * A : ethanol, E : diethyl ether, H : hexane

Example 17

2-Diethylaminoethyl α-benzyl-1,2-benzisoxazole-3-acetate fumalate:

p-Toluenesulfonyl chloride (1.43 g) was added at 0°C to a solution of α-benzyl-1,2-benzisoxazole-3-acetic acid (1 g) in pyridine (20 ml), and the mixture was stirred for 15 min at 0°C. Diethylaminoethanol (0.44 g) was added to the mixture. After stirring for 1 hr at room temperature, the reaction mixture was diluted with water and extracted with dichloromethane. The organic layer was dried over anhydrous sodium sulfate and evaporated *in vacuo*. The residue was applied on a column of silica gel. The 5% methanol-chloroform eluate gave an oil (0.58 g) which was converted into fumalate according to a usual method. Recrystallization from a mixture of ethanol and hexane gave 2-diethylaminoethyl α-benzyl-1,2-benzisoxazole-3-acetate fumalate (0.5 g), m.p. 123-127°C.

Example 18

1-Ethyl-3-piperidyl α-benzyl-1,2-benzisoxazole-3-acetate fumalate:

In substantially the same manner as in Example 17, using 1-ethyl-3-hydroxypiperidine instead of diethylaminoethanol, 1-ethyl-3-piperidyl α-benzyl-1,2-benzisoxazole-3-acetate fumalate, m.p. 124-136°C (dec.) recrystallized from a mixture of ethanol and hexane, was obtained.

Example 19

3-Diethylaminopropyl α-hydroxy-1,2-benzisoxazole-3-acetate oxalate:

A solution of α-hydroxy-1,2-benzisoxazole-3-acetic acid (2.45 g) and 3-diethylaminopropyl chloride (1.9 g) in isopropyl alcohol (15 ml) was heated with stirring to distill off isopropyl alcohol (10 ml). The residual mixture was heated at 90°C for 6 hr. After being cooled, toluene and 5% hydrochloric acid were added to the reaction mixture. After shaking the mixture, the aqueous layer was made alkaline by addition of concentrated ammonium hydroxide and extracted with toluene. The toluene layer was dried over anhydrous sodium sulfate and evaporated to give an oily 3-diethylaminopropyl α-hydroxy-1,2-benzisoxazole-3-acetate (2.5 g) which was converted into oxalate in a manner similar to Example 2. Recrystallization from a mixture of ethanol and diethyl ether gave 3-diethylaminopropyl α-hydroxy-1,2-benzisoxazole-3-acetate oxalate, m.p. 94-96°C.

Examples 20 to 26

Various compounds listed in the following Table 5 were prepared in substantially the same manner as in Example 19, using the corresponding α-substituted-1,2-benzisoxazole-3-acetic acid and aminoalkyl chloride.

Table 5

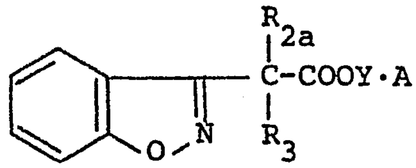

| Ex. | $R_{2a}$ | $R_3$ | Y | A | m.p. (°C) Recry. Solv.* |
|---|---|---|---|---|---|
| 20 | OH | H | $-(CH_2)_2N(C_2H_5)_2$ | HBr | 124-126 (IP-E) |
| 21 | " | " | $-(CH_2)_2$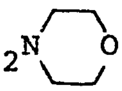 | " | 148-150 (IP-E) |
| 22 | " | " | $-(CH_2)_3N(CH_3)_2$ | " | 102-104 (IP-E) |
| 23 | " | " | $-(CH_2)_2$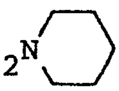 | " | 138-140 (IP-E) |
| 24 | " | $-CH_2Ph$ | $-(CH_2)_2N(C_2H_5)_2$ | " | 145-147 (EA-H) |
| 25 | " | " | $-(CH_2)_3N(C_2H_5)_2$ | --- | oil ** |
| 26 | $-CH_2OH$ | H | $-(CH_2)_2N(C_2H_5)_2$ | HBr | oil *** |

Ph : $-C_6H_5$

* IP : isopropyl alcohol, E : diethyl ether
EA : ethyl acetate, H : hexane

** Molecular ion peak ($M^+$) in the mass spectrum : m/z 396

*** Molecular ion peak ($M^+$) in the mass spectrum : m/z 306

Example 27

2-Diethylaminoethyl α,α-dibenzyl-1,2-benzisoxazole-3-acetate oxalate:

A mixture of α,α-dibenzyl-1,2-benzisoxazole-3-acetic acid (1.0 g) and thionyl chloride (10 ml) was refluxed for 2 hr and then evaporated in vacuo. A solution of diethylaminoethanol (0.49 g) in chloroform (20 ml) was added to the residue. The mixture was stirred for 2 hr at room temperature and then refluxed for 5 hr. The reaction mixture was washed with diluted hydrochloric acid and 10 % sodium carbonate in turn. The organic layer was dried over anhydrous sodium sulfate and evaporated. The residue was applied on a column of silica gel. The 3 % methanol-chloroform eluate gave oily 2-diethylaminoethyl α,α-dibenzyl-1,2-benzisoxazole-3-acetate (0.73 g) which was converted into oxalate in a manner similar to Example 2. Recrystallization from a mixture of ethanol and diethyl ether gave 2-diethylaminoethyl α,α-dibenzyl-1,2-benzisoxazole-3-acetate oxalate (0.6 g), m.p. 102-108°C.

Example 28 to 30

Various compounds listed in Table 6 were prepared in substantially the same manner as in Example 27, using the corresponding α,α-dibenzyl-1,2-benzisoxazole-3-acetic acid and aminoalcohol.

 

Table 6

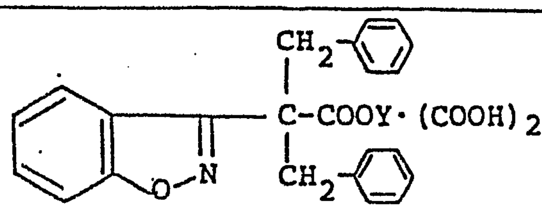

| Ex. | Y | m.p. (°C) | Recry. Solv.* |
|---|---|---|---|
| 28 | 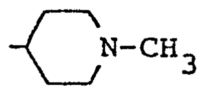 | 220-225 | (A-E) |
| 29 | 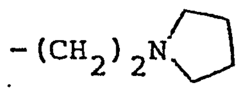 | 142-143 | (A-E) |
| 30 | $-(CH_2)_2N(CH_3)_2$ | 171-173 | (A-E) |

* A : ethanol, E : diethyl ether

Example 31

1-Ethyl-1-methyl-4-[2-(1,2-benzisoxazol-3-yl)-2-cyclohexylacetoxy]piperidinium bromide:

Ethyl bromide (1.6 g) was added to a solution of 1-methyl-4-piperidyl α-cyclohexyl-1,2-benzisoxazole-3-acetate (1.1 g) in acetone (10 ml). The mixture was allowed to stand at room temperature for 2 days. The resulting crystals were collected by filtration and recrystallized from a mixture of ethanol and diethyl ether to give 1-ethyl-1-methyl-4-[2-(1,2-benzisoxazol-3-yl)-2-cyclohexylacetoxy]piperidinium bromide (0.5 g), m.p. 176-180°C.

Example 32 to 35

The various compounds listed in Table 7 were prepared in substantially the same manner as in Example 31, using the corresponding α-cyclohexyl-1,2-benzisoxazole-3-acetic acid esters and methyl iodide.

Table 7

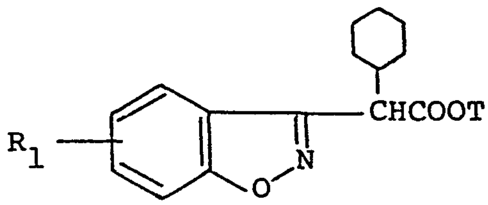

| Ex. | $R_1$ | T | m.p. (°C) | Recry. Solv.* |
|---|---|---|---|---|
| 32 | H | $-(CH_2)_3\underset{CH_3}{\overset{+}{N}}(C_2H_5)_2\ I^-$ | 88-91 | (A) |
| 33 | " | 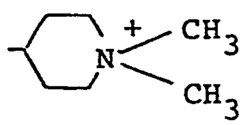 $I^-$ | 103-104 | (THF) |
| 34 | " | $-(CH_2)_2\underset{CH_3}{\overset{+}{N}}(C_2H_5)_2\ I^-$ | 118-124 | (A-E) |
| 35 | 5-Cl | 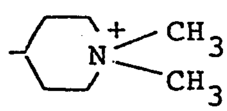 $I^-$ | 129-131 | (AC-E) |

* A : ethanol, THF : tetrahydrofran
E : diethyl ether, AC : acetone

Example 36

| | per 1,000 tablets |
|---|---|
| 3-Diethylaminopropyl α-cyclohexyl-1,2-benzisoxazole-3-acetate hydrobromide .... | 20 g |
| Corn starch ................................ | 28 g |
| Lactose .................................... | 65 g |
| Microcrystalline cellulose ................. | 30 g |
| Hydroxypropyl cellulose .................... | 5 g |
| Light anhydrous silicic acid ............... | 1 g |
| Magnesium stearate ......................... | 1 g |

The above components were blended, granulated and made into tablets by a conventional method to form 1,000 tablets, each weighting 150 mg.

Example 37

| | per 1,000 capsules |
|---|---|
| 3-Diethylaminopropyl α-cyclohexyl-1,2-benzisoxazole-3-acetate hydrobromide .... | 40 g |
| Corn starch ................................ | 80 g |
| Lactose .................................... | 56 g |
| Microcrystalline cellulose ................. | 40 g |
| Talc ....................................... | 2 g |
| Magnesium stearate ......................... | 2 g |

The above components were blended, granulated and filled into 1,000 capsules by a conventional method.

What is claimed is:

1. A 1,2-benzisoxazole compound of the formula:

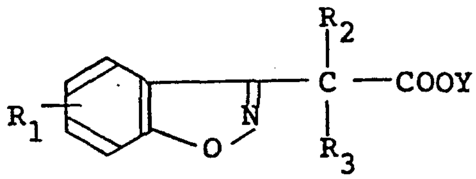

wherein $R_1$ is hydrogen atom, a halogen atom, hydroxy group, a lower alkyl group, trifluoromethyl group, or a lower alkoxy group, $R_2$ is hydroxy group, hydroxymethyl group, a cycloalkyl group, phenoxy group, phenylthio group, or benzyl group, $R_3$ is hydrogen atom or benzyl group, Y is a group of the formula: 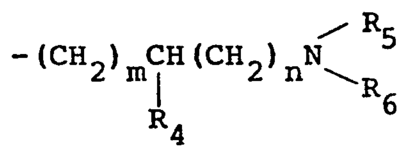 or 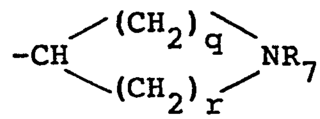 , $R_4$ is hydrogen atom or a lower alkyl group, $R_5$ and $R_6$ are the same or different and are each a lower alkyl group or combine together with the adjacent nitrogen atom to form a heterocyclic group, $R_7$ is a lower alkyl group, m and n are the same or different and are each an integer of 0 to 3, sum of m and n is an integer of 1 to 4, and q and r are the same or different and are each an integer of 1 to 3, and sum of q and r is an integer of 3 to 5, and a pharmaceutically acceptable acid addition salt or quaternary ammonium salt thereof.

2. A compound according to claim 1, wherein $R_1$ is hydrogen atom, a halogen atom or a lower alkyl group.

3. A compound according to claim 1, wherein $R_1$ is hydrogen atom or a halogen atom.

4. A compound according to claim 1, whrerin $R_2$ is a cycloalkyl group.

5. A 1,2-benzisoxazole compound of the formula:

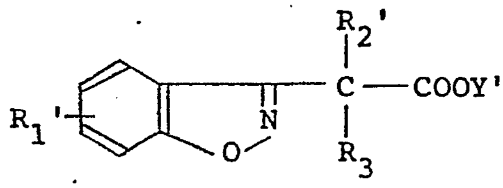

wherein $R_1'$ is hydrogen atom, a halogen atom or a lower alkyl group, $R_2$, is cyclohexyl group, benzyl group or phenylthio group, Y' is a group of the formula:

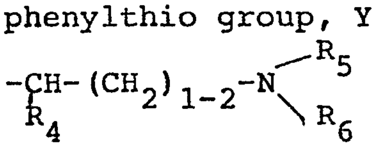 or 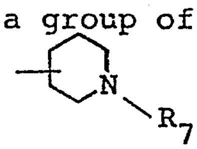 , and $R_3$, $R_4$, $R_5$, $R_6$ are the same as defined in claim 1, and a pharmaceutically acceptable acid addition salt thereof.

6. A compound according to claim 5, whrerin $R_2'$ is benzyl group and $R_3$ is benzyl group.

7. 3-Diethylaminopropyl α-cyclohexyl-1,2-benzisoxazole-3-acetate and a pharmaceutically acceptable acid addition salt thereof.

8. A quaternary ammonium salt of the formula:

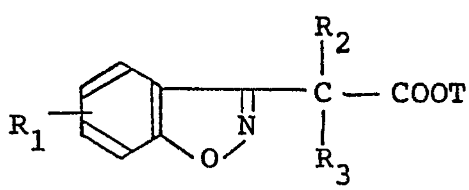

wherein T is a group of the formula:

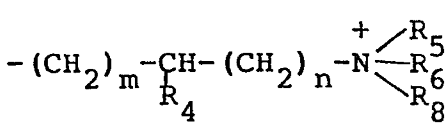 $X^-$ or 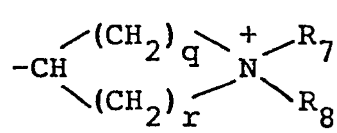 $X^-$

$R_8$ is a lower alkyl group, $X^-$ is a pharmaceutically acceptable anion, and $R_1$, $R_2$, $R_3$, $R_4$, $R_5$, $R_6$, $R_7$, m, n, q and r are the same as defined in claim 1.

9. A quaternary ammonium salt according to claim 8, wherein $R_1$ is hydrogen atom, a halogen atom or a lower alkyl group.

10. A quaternary ammonium salt according to claim 8, wherein $R_1$ is hydrogen atom or a halogen atom.

11. A quaternary ammonium salt according to claim 8, wherein $R_2$ is a cycloalkyl group.

12. A quaternary ammonium salt of the formula

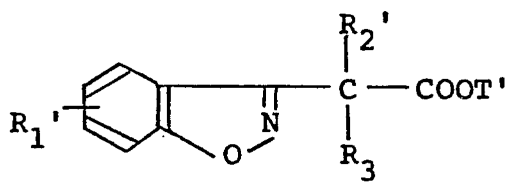

wherein T' is a group of the formula: 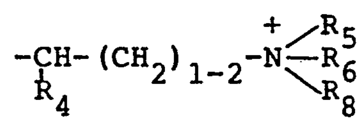 $X^-$

or 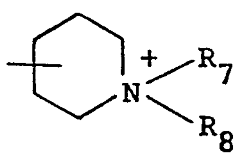 $X^-$ , and $R_1'$, $R_2'$, $R_3$, $R_4$, $R_5$, $R_6$, $R_7$, $R_8$ and $X^-$ are the same as defined above.

13. A pharmaceutical composition comprising an effective amount of a 1,2-benzisoxazole derivatives as set forth in claim 1, or a pharmaceutically acceptable acid addition salt thereof in admixture with a pharmaceutically acceptable carrier.

14. A pharmaceutical composition according to claim 13, wherein the 1,2-benzisoxazole derivative is 3-diethylaminopropyl α-cyclohexyl-1,2-benzisoxazole-3-acetate.

15. A pharmaceutical composition comprising an effective amount of a quaternary ammonium salt set forth in claim 8 in admixture with a pharmaceutically acceptable carrier.